# EUROPEAN PATENT APPLICATION

(11) **EP 4 678 635 A1**
(43) Date of publication of application: **14.01.2026**
(21) Application number: 24766454.3
(22) Date of filing: 06.03.2024
(51) Int. Cl.: C07D 233/88, A61K 31/4164, A61P 35/00

(54) **CRYSTAL FORM OF NIROGACESTAT DIHYDROBROMATE, AND PREPARATION METHOD THEREFOR AND USE THEREOF**

(30) Priority: 09.03.2023 CN 202310224619; 16.03.2023 CN 202310257267
(71) Applicant: Crystal Pharmaceutical (Suzhou) Co., Ltd., Suzhou, Jiangsu 215123 (CN)
(72) Inventor: SHI, Jiaming, Suzhou, Jiangsu 215123 (CN); ZHANG, Jing, Suzhou, Jiangsu 215123 (CN); LI, Yingjie, Suzhou, Jiangsu 215123 (CN); HUANG, Chunxiang, Suzhou, Jiangsu 215123 (CN); WU, Kelin, Suzhou, Jiangsu 215123 (CN); MENG, Liping, Suzhou, Jiangsu 215123 (CN)
(74) Representative: dompatent
(86) International application number: PCT/CN2024/080216
(87) International publication number: WO 2024/183739

(57) **Abstract**

Novel crystalline forms of Nirogacestat dihydrobromide and preparation methods thereof, pharmaceutical compositions containing the crystalline forms, and uses of the crystalline forms for preparing γ-secretase inhibitor drugs and drugs for treating desmoid tumors.

## Description

### TECHNICAL FIELD

The present disclosure pertains to the field of chemical crystallography, particularly relates to novel crystalline forms of Nirogacestat dihydrobromide, preparation method and use thereof.

### BACKGROUND

Desmoid tumors, also known as aggressive fibromatosis, are rare locally invasive, slow growing soft tissue tumors. Although considered benign because of their inability to metastasize, desmoid tumors can cause significant morbidity and occasionally mortality in patients, thus creating an urgent need for effective drug treatments.

A promising development is the γ-secretase inhibitor Nirogacestat, developed by SpringWorks Therapeutics, which has been approved for marketing in the US, and is used in the treatment of desmoid tumors. Nirogacestat is being investigated for the treatment of desmoid tumors due to its ability to bind to γ-secretase, blocking proteolytic activation of Notch receptors and obtained positive clinical results. Previous clinical study data have shown that Notch signaling plays an important role in cancer development. Hence, inhibiting Notch signaling is an important strategy for treating desmoid tumors.

The chemical name of Nirogacestat is (S)-2-(((S)-6,8-difluoro-1,2,3,4-tetrahydronaphthalen-2-yl)amino)-N-(1-(2-methyl-1-(neopentylamino) propan-2-yl)-1H-imidazol-4-yl) pentanamide (referred to as "Compound I") and the structure of Compound I dihydrobromide is shown as the follows:

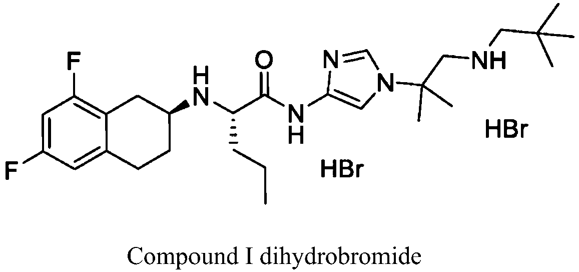

It is well known in the field that drug polymorphism is a common phenomenon in small molecule drug development and it is an important factor affecting drug quality. A crystalline form is a solid material whose constituents are arranged in a highly ordered microscopic structure, forming a crystal lattice that extends in all directions. Polymorphism refers to the phenomenon that a compound exists in more than one crystalline form. Compounds may exist in one or more crystalline forms, but their existence and characteristics cannot be predicted with any certainty.

Different crystalline forms of an active pharmaceutical ingredient (API) have different physicochemical properties, such as chemical stability and solubility, which can affect drug's in vivo dissolution and absorption and will further affect drug's clinical efficacy and safety to some extent. In addition, different solid forms of APIs may have different manufacturability characteristics, including compressibility, milling behavior, and stability under compression pressure during tableting, which can impact the processing and handling during drug production. Therefore, different solid forms of APIs may possess unique properties, offering opportunities to improve the performance of pharmaceutical products.

Prior art WO2021029854A1 disclosed multiple crystalline forms of Compound I dihydrobromide. Example 6 of WO2021029854A1 indicates that Form A is the most stable and preferred crystalline form. However, the inventors of the present disclosure have found that Form A exhibits low solubility.

To explore novel solid forms that could improve the drug performance, the inventors of the present disclosure surprisingly obtained novel crystalline forms of Compound I dihydrobromide of the present disclosure, which have advantages in at least one aspect of solubility, hygroscopicity, purification ability, stability, adhesiveness, compressibility, flowability, in vitro and in vivo dissolution, and bioavailability, etc. In particular, the crystalline forms of the Compound I dihydrochloride of the present disclosure have advantages such as good solubility, high density, good compressibility and good stability, which solve the problems existing in prior arts and are of great significance for the development of drugs containing Compound I dihydrobromide.

### SUMMARY

The present disclosure is to provide novel crystalline forms of Compound I dihydrobromide, preparation method and pharmaceutical compositions comprising the crystalline forms.

According to the objective of the present disclosure, crystalline form CSV of Compound I dihydrobromide is provided by the present disclosure (hereinafter referred to as Form CSV).

In one aspect provided herein, the X-ray powder diffraction pattern of Form CSV comprises characteristic peaks at 2theta values of 6.1°±0.2°, 9.6°±0.2° and 13.6°±0.2° using CuKα radiation.

Furthermore, the X-ray powder diffraction pattern of Form CSV comprises one or two or three characteristic peaks at 2theta values of 4.5°±0.2°, 16.1°±0.2° and 18.6°±0.2° using CuKα radiation. Preferably, the X-ray powder diffraction pattern of Form CSV comprises characteristic peaks at 2theta values of 4.5°±0.2°, 16.1°±0.2° and 18.6°±0.2° using CuKα radiation.

Furthermore, the X-ray powder diffraction pattern of Form CSV comprises one or two or three characteristic peaks at 2theta values of 12.7°±0.2°, 21.5°±0.2° and 23.0°±0.2° using CuKα radiation. Preferably, the X-ray powder diffraction pattern of Form CSV comprises characteristic peaks at 2theta values of 12.7°±0.2°, 21.5°±0.2° and 23.0°±0.2° using CuKα radiation.

In another aspect provided herein, the X-ray powder diffraction pattern of Form CSV comprises three or four or five or six or seven or eight or nine characteristic peaks at 2theta values of 6.1°±0.2°, 9.6°±0.2°, 13.6°±0.2°, 4.5°±0.2°, 16.1°±0.2°, 18.6°±0.2°, 12.7°±0.2°, 21.5°±0.2° and 23.0°±0.2° using CuKα radiation.

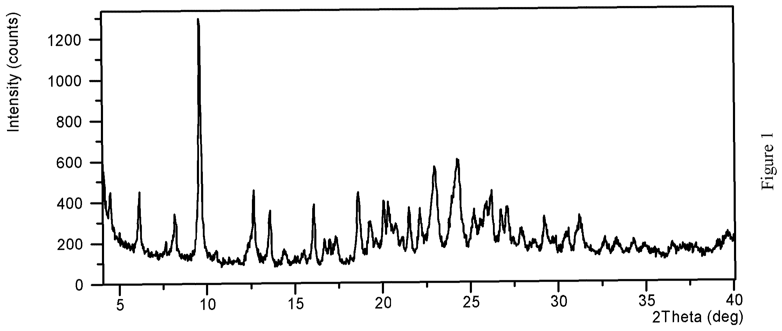

Without any limitation being implied, an XRPD pattern of Form CSV is substantially as depicted in Figure 1.

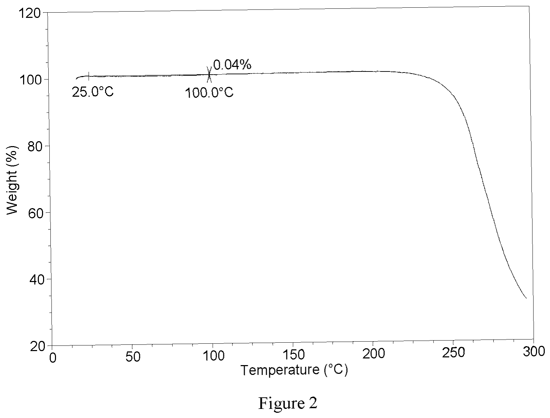

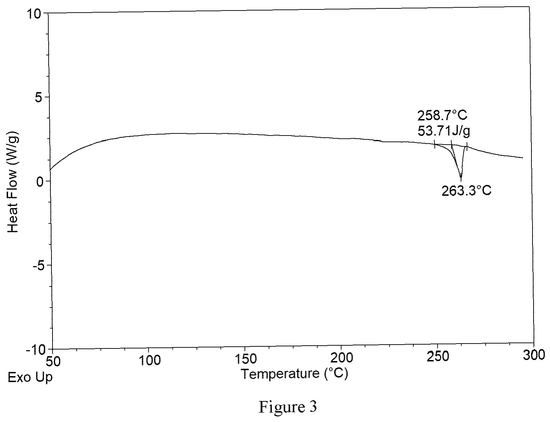

Without any limitation being implied, a TGA curve of Form CSV is substantially as depicted in Figure 2, which shows 0.04% weight loss when heated to about 100 °C. Without any limitation being implied, a DSC curve of Form CSV is substantially as depicted in Figure 3, which shows an endothermic peak with an onset temperature at about 259 °C and a peak temperature at about 263 °C.

Without any limitation being implied, Form CSV is an anhydrate.

According to the objective of the present disclosure, a process for preparing Form CSV is also provided. The process comprises:
Dissolving Compound I dihydrobromide in a mixed solvent of an ether and an acid, evaporating to obtain a solid, then heating the obtained solid to get Form CSV.

Form CSV of the present disclosure exhibits the following unexpected technical effects:
(1) Compared with the prior art, Form CSV of the present disclosure has better compressibility. Failure in hardness/friability test and tablet crack issue can be avoided due to good compressibility of Form CSV, making the preparation process more reliable, improving product appearance, promoting product quality and production efficiency.
(2) Compared with the prior art, Form CSV of the present disclosure has higher density. Experimental results indicate that the bulk and tapped density of Form CSV are superior to those of the prior art Form A. Higher density of Form CSV is beneficial to large-scale production. Higher density of Form CSI can also reduce dust, reduce occupational hazard.
(3) Form CSV of the present disclosure has good stability.

Crystalline form of Form CSV doesn't change for at least 6 months when stored under conditions of 25 °C/60%RH and 40 °C/75%RH. And the chemical purity remains substantially unchanged during storage. Crystalline form of Form CSV doesn't change for at least 2 months when stored under the condition of 60 °C/75%RH. And the chemical purity remains substantially unchanged during storage. These results indicate that Form CSV has good stability under long-term, accelerated and stress conditions. Form CSV has good stability under mechanical force. The crystalline form of Form CSV remains unchanged after milling and under varying compression forces.

High humidity conditions caused by seasonal variations, regional climate differences, and environmental factors can affect the storage, transportation, and manufacturing of APIs. In addition, grinding or milling of APIs is often required during formulation processing. Form CSV has good stability, which helps avoid the impact on drug quality due to crystal transformation during storage, transportation, and manufacturing. Besides, it reduces the risk of decreased crystalline and undesired polymorphic transitions during formulation processing. As a result, it ensures consistent and controllable quality of APIs, minimizes quality fluctuations, bioavailability changes and toxicity caused by crystal transformation.

According to the objective of the present disclosure, crystalline form CSVI of Compound I dihydrobromide is provided by the present disclosure (hereinafter referred to as Form CSVI).

In one aspect provided herein, the X-ray powder diffraction pattern of Form CSVI comprises characteristic peaks at 2theta values of 8.8°+0.2°, 10.4°±0.2°, 19.4°±0.2° and 20.9°±0.2° using CuKα radiation.

Furthermore, the X-ray powder diffraction pattern of Form CSVI comprises one or two or three characteristic peaks at 2theta values of 22.9°±0.2°, 26.8°±0.2° and 25.9°±0.2° using CuKα radiation. Preferably, the X-ray powder diffraction pattern of Form CSVI comprises characteristic peaks at 2theta values of 22.9°±0.2°, 26.8°±0.2° and 25.9°±0.2° using CuKα radiation.

In another aspect provided herein, the X-ray powder diffraction pattern of Form CSVI comprises four or five or six or seven or eight or nine characteristic peaks at 2theta values of 8.8°+0.2°, 10.4°+0.2°, 19.4°+0.2°, 20.9°+0.2°, 22.9°+0.2°, 26.8°+0.2°, 25.9°+0.2°, 6.3°+0.2° and 12.7°+0.2° using CuKα radiation.

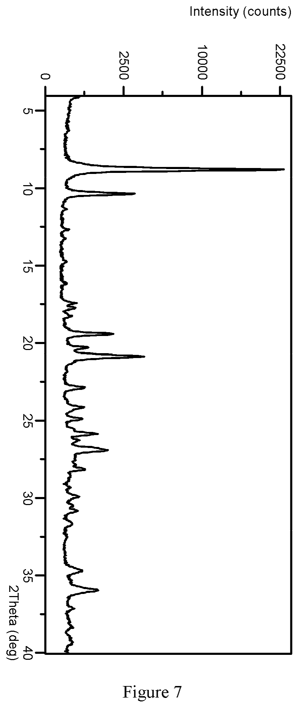

Without any limitation being implied, an XRPD pattern of Form CSVI is substantially as depicted in Figure 7 or Figure 8 or Figure 10.

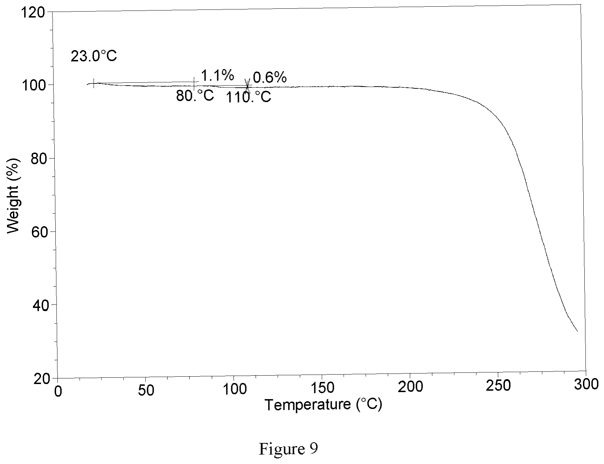

Without any limitation being implied, a TGA curve of Form CSVI is substantially as depicted in Figure 9, which shows 1.1% weight loss when heated to about 80 °C and 0.6% weight loss when heated from 80 °C to 110 °C.

Without any limitation being implied, Form CSVI is a hydrate, preferably a 0.25 hydrate.

According to the objective of the present disclosure, a process for preparing Form CSVI is also provided. The process comprises:
Dissolving Compound I dihydrobromide in an alcohol, filtering the solution, adding toluene to the filtrate, standing at -20 °C to precipitate a solid, isolating the solid, and vacuum drying with water compensation to obtain Form CSVI.

Furthermore, said alcohol is preferably methanol. Said volume ratio of alcohol to toluene is preferably 1:5. Said drying temperature is preferably from room temperature to 80 °C.

Form CSVI of the present disclosure exhibits the following unexpected technical effects:
(1) Compared with the prior art, Form CSVI of the present disclosure has better solubility. Nirogacestat is a poorly water-soluble drug and belongs to BCS Class II. Compared to the prior art Form A, Form CSVI shows higher solubility in FaSSIF and FeSSIF, which is beneficial for enhancing drug absorption in the human body and improving bioavailability.
(2) Compared with the prior art, Form CSVI of the present disclosure has better compressibility. Failure in hardness/friability test and tablet crack issue can be avoided due to good compressibility of Form CSVI, making the preparation process more reliable, improving product appearance, promoting product quality and production efficiency.
(3) Form CSVI of the present disclosure has good stability.

Crystalline form of Form CSVI remains unchanged for at least 8 months when stored under conditions of 25 °C/60%RH and 40 °C/75%RH, and for at least 2 months when stored under the condition of 60 °C/75%RH. These results indicate that Form CSVI has good stability under long-term, accelerated and stress conditions.

Form CSVI has good stability under mechanical force. The crystalline form of Form CSVI remains unchanged after milling and under varying compression forces.

High humidity conditions caused by seasonal variations, regional climate differences, and environmental factors can affect the storage, transportation, and manufacturing of APIs. In addition, grinding or milling of APIs is often required during formulation processing. Form CSVI has good stability, which helps avoid the impact on drug quality due to crystal transformation during storage, transportation, and manufacturing. Besides, it reduces the risk of decreased crystalline and undesired polymorphic transitions during formulation processing. As a result, it ensures consistent and controllable quality of APIs, minimizes quality fluctuations, bioavailability changes and toxicity caused by crystal transformation.

According to the objective of the present disclosure, the present disclosure provides a pharmaceutical composition comprising a therapeutically effective amount of Form CSV, or Form CSVI, or combinations thereof, and pharmaceutically acceptable excipients.

According to the objective of the present disclosure, the present disclosure provides use of Form CSV, or Form CSVI, or combinations thereof for preparing drugs of γ-secretase inhibitor.

According to the objective of the present disclosure, the present disclosure provides use of Form CSV, or Form CSVI, or combinations thereof for preparing drugs for treating desmoid tumors.

### BRIEF DESCRIPTION OF THE DRAWINGS

Figure 1 shows an XRPD pattern of Form CSV.
Figure 2 shows a TGA curve of Form CSV.
Figure 3 shows a DSC curve of Form CSV.
Figure 4 shows an XRPD pattern overlay of Form CSV before and after storage under different conditions (from top to bottom: initial, 25 °C/60%RH for 6 months, 40 °C/75%RH for 6 months, 60 °C/75%RH for 2 months).
Figure 5 shows an XRPD pattern overlay of Form CSV before and after tableting (from top to bottom: before tableting, after tableting at 5 kN, 10 kN, and 20 kN).
Figure 6 shows an XRPD pattern overlay of Form CSV before and after ball milling (from top to bottom: before ball milling, after ball milling).
Figure 7 shows an XRPD pattern of Form CSVI.
Figure 8 shows an XRPD pattern of Form CSVI.
Figure 9 shows a TGA curve of Form CSVI.
Figure 10 shows an XRPD pattern of Form CSVI.
Figure 11 shows an XRPD pattern overlay of Form CSVI before and after storage under different conditions (from top to bottom: initial, 25 °C/60%RH for 8 months, 40 °C/75%RH for 8 months, 60 °C/75%RH for 2 months).
Figure 12 shows an XRPD pattern overlay of Form CSVI before and after tableting (from top to bottom: before tableting, after tableting at 5 kN, 10 kN, and 20 kN).
Figure 13 shows an XRPD pattern overlay of Form CSVI before and after ball milling (from top to bottom: before ball milling, after ball milling).

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.

The abbreviations used in the present disclosure are explained as follows:
XRPD: X-ray Powder Diffraction
DSC: Differential Scanning Caborimetry
TGA: Thermo Gravimetric Analysis
¹H NMR: Proton Nuclear Magnetic Resonance
HPLC: High Performance Liquid Chromatography
FeSSIF: Fed State Simulated Intestinal Fluid
FaSSIF: Fasted State Simulated Intestinal Fluid
RH: Relative Humidity
RT: Room Temperature

Instruments and methods used for data collection:
XRPD patterns in the present disclosure were acquired by a Bruker D8 ADVANCE X-ray powder diffractometer. The parameters of the X-ray powder diffraction method are as follows:
   X-Ray source: Cu, Kα
   Kα1 (Å): 1.54060; Kα2 (Å): 1.54439
   Kα2/Kα1 intensity ratio: 0.50
   Voltage: 40 kV
   Current: 40 mA
   Scan range (2θ): from 4.0 degree to 40.0 degree
DSC data in the present disclosure were acquired by a TA Q2000. The parameters of the DSC method of the present disclosure are as follows:
   Heating rate: 10 °C/min
   Purge gas: N₂
TGA data in the present disclosure were acquired by a TA Q500. The parameters of the TGA method of the present disclosure are as follows:
   Heating rate: 10 °C/ min
   Purge gas: N₂

¹H NMR data in the present disclosure were collected from a Bruker Avance II DMX 400M HZ NMR spectrometer. 1-5 mg of sample was weighed and dissolved with 0.5 mL of deuterated dimethyl sulfoxide to obtain a solution with a concentration of 2-10 mg/mL.

The parameters of related substance detection in the present disclosure are shown in Table 1.

**Table 1**

| | | |
|---|---|---|
| Instrument | Agilent 1260 | |
| Column | ZORBAX Eclipse XDB-C18, 4.6×100 mm 3.5 µm | |
| Ghost-Buster column | Welch Ghost-Buster, 4.6×50 mm | |
| Mobile phase | A: 10 mM KH₂PO₄ in H₂O (pH 6.0, Triethylamine) | |
| | B: Acetonitrile | |
| Gradient | Time (min) | % A |
| | 0.0 | 80 |
| | 1.0 | 80 |
| | 6.0 | 50 |
| | 19.0 | 20 |
| | 30.0 | 20 |
| | 31.0 | 80 |
| | 40.0 | 80 |
| Run time | 40.0 min | |
| Flow rate | 1.0 mL/min | |
| Injection volume | 5 µL | |
| Detector wavelength | 240 nm | |
| Column temperature | 40 °C | |
| Sample pan temperature | RT | |
| Diluent | 50% Acetonitrile | |

The parameters of kinetic solubility in the present disclosure are shown in Table 2.

**Table 2**

| | | |
|---|---|---|
| Instrument | Agilent 1260 with DAD detector | |
| Column | ZORBAX Eclipse XDB-C18, 4.6×100 mm 3.5µm | |
| Ghost-Buster column | Welch Ghost-Buster, 4.6×50 mm | |
| Mobile phase | A: 0.1%H₃PO₄ in H₂O (pH 6.0, Triethylamine) | |
| | B: Acetonitrile | |
| Gradient | Time (min) | % B |
| | 0.0 | 78 |
| Run time | 8.0 min | |
| Post time | 0.0 min | |
| Flow rate | 1.0 mL/min | |
| Injection volume | 5 µL | |
| Detector wavelength | 240 nm | |
| Column temperature | 40 °C | |
| Sampler temperature | RT | |
| Diluent | 50% Acetonitrile | |

Said "evaporating" is accomplished by using a conventional method in the field such as slow evaporation or rapid evaporation. Slow evaporation is accomplished in a container covered by a sealing film with pinholes. Rapid evaporation is accomplished in an open container.

Said "vacuum drying with water compensation" is accomplished by using a conventional method in the field. The drying temperature can be room temperature or higher, preferably from room temperature to about 60 °C, or up to 50 °C, or up to 40 °C. The drying time can range from 2 to 48 hours, or overnight. Vacuum drying with water compensation is typically conducted in a vacuum oven with a cup of water placed inside.

Said "characteristic peak" refers to a representative diffraction peak used to distinguish crystals, which usually can have a deviation of ±0.2° using CuKα radiation.

Said "anhydrate" refers to a solid form that without crystalline water or solvents. Said "hydrate" refers to a solid form with crystalline water.

In the present disclosure, "crystal" or "crystalline form" refers to the crystal or the crystalline form being identified by the X-ray diffraction pattern shown herein. Those skilled in the art are able to understand that the X-ray powder diffraction pattern depends on the instrument conditions, the sample preparation and the purity of samples. The relative intensity of the diffraction peaks in the X-ray diffraction pattern may also vary with the experimental conditions. Therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CSV or CSVI of the present disclosure is pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

Unless otherwise specified, the following examples were conducted at room temperature. The term "room temperature" does not refer to a specific temperature value, but rather to a temperature range of 10-30 °C.

According to the present disclosure, Compound I used as raw materials include, but are not limited to solid (crystalline and amorphous), oil, liquid form or solution. Preferably, Compound I used as the raw material is a solid.

Raw materials of Compound I used in the following examples were prepared by prior arts, for example, the method disclosed in WO2021029854A1.

### Example 1 Preparation of Form CSV

Approximately 85 mg of Compound I dihydrobromide solid was weighted into a vial. 2.0 mL of a mixed solvent of 2-methyltetrahydrofuran/formic acid (1:1, v/v) was added. A clear solution was obtained after dissolution and filtration. The obtained solution was evaporated at room temperature for about 3 days to obtain a solid. The obtained solid was heated to 160 °C under nitrogen protection, maintained at 160 °C for 10 minutes, and then cooled to room temperature. It was then heated to 200 °C under nitrogen protection, maintained at 200 °C for 60 minutes, and cooled to room temperature to obtain a crystalline solid.

The obtained crystalline solid was confirmed as Form CSV. The XRPD pattern is substantially as depicted in Figure 1, and the XRPD data are listed in Table 3.

The TGA curve of Form CSV is depicted in Figure 2, which shows Form CSV has an approximately weight loss of 0.04% when heated to 100 °C.

The DSC curve of Form CSV is depicted in Figure 3. It shows an endothermic peak with an onset temperature of about 259 °C and a peak temperature of about 263 °C.

**Table 3**

| 20 (°) | d-spacing (Å) | Relative intensity (%) |
|---|---|---|
| 4.5 | 19.6 | 29.1 |
| 6.1 | 14.4 | 29.2 |
| 8.2 | 10.8 | 19.3 |
| 9.6 | 9.2 | 100.0 |
| 10.5 | 8.4 | 4.3 |
| 12.7 | 7.0 | 29.4 |
| 13.6 | 6.5 | 21.2 |
| 14.4 | 6.1 | 4.9 |
| 15.5 | 5.7 | 4.5 |
| 16.1 | 5.5 | 23.3 |
| 16.7 | 5.3 | 8.7 |
| 17.3 | 5.1 | 9.6 |
| 18.6 | 4.8 | 28.2 |
| 19.3 | 4.6 | 15.4 |
| 20.1 | 4.4 | 24.9 |
| 20.3 | 4.4 | 21.8 |
| 20.8 | 4.3 | 14.5 |
| 21.5 | 4.1 | 21.7 |
| 22.1 | 4.0 | 20.4 |
| 23.0 | 3.9 | 37.9 |
| 24.3 | 3.7 | 40.1 |
| 25.2 | 3.5 | 18.2 |
| 26.2 | 3.4 | 25.7 |
| 26.8 | 3.3 | 19.0 |
| 27.1 | 3.3 | 18.9 |
| 27.9 | 3.2 | 11.9 |
| 28.6 | 3.1 | 6.9 |
| 29.2 | 3.1 | 15.2 |
| 30.5 | 2.9 | 8.7 |
| 31.2 | 2.9 | 15.9 |
| 32.7 | 2.7 | 6.6 |
| 33.3 | 2.7 | 6.5 |
| 34.3 | 2.6 | 6.1 |
| 36.5 | 2.5 | 4.4 |

### Example 2 Compressibility of Form CSV

Tablets were prepared by an ENERPAC manual tablet press. About 60 mg of Form CSV and the prior art Form A were separately added to Φ6mm round tooling punches and compressed into tablets under a pressure of 3 kN. The tablets were left at RT for 24 h to allow complete elastic recovery. The diameter (D) and thickness (L) were measured using a vernier caliper, and their radial crushing strength (hardness, H) was determined using a tablet hardness tester. The tensile strength (T) of the powders was calculated with the following formula: T=2H/πDL. The test results show that the average tensile strength of Form CSV was 0.55 MPa, while that of the prior art Form A was 0.37 MPa. Under a certain force, higher tensile strength indicates better compressibility. The results indicate Form CSV exhibits superior compressibility compared to the prior art Form A.

### Example 3 Density of Form CSV

About 500 mg of sample was gently loaded into a 5-mL graduated cylinder to measure the bulk volume before tapping. The sample was then tapped 1250 times on a ZS-2E tap density tester to achieve a tapped state, and the tapped volume was recorded. The bulk density ρ0 and tapped density ρf were calculated accordingly. The density results of Form CSV and the prior art Form A are listed in Table 4, indicating that the density of Form CSV is superior to that of the prior art Form A.

**Table 4**

| Crystalline form | Bulk density (g/mL) | Tapped density (g/mL) |
|---|---|---|
| The prior art Form A | 0.251 | 0.282 |
| Form CSV | 0.422 | 0.506 |

### Example 4 Stability of Form CSV

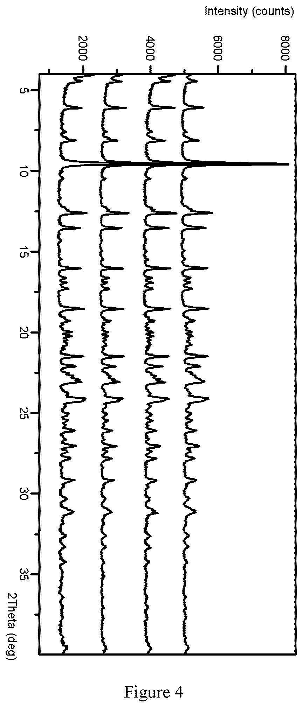

An appropriate amount of Form CSV was packaged in a sealed container and then stored under the conditions of 25 °C/60%RH, 40 °C/75%RH and 60 °C/75%RH, respectively. Crystalline form and purity were analyzed by XRPD and HPLC. The results are listed in Table 5, and the XRPD pattern overlay is depicted in Figure 4. The results show that Form CSV remains stable for at least 6 months under 25 °C/60%RH and 40 °C/75%RH conditions, indicating Form CSV has good stability under long-term and accelerated conditions. Meanwhile, Form CSV remains stable for at least 2 months under 60 °C/75%RH condition, indicating Form CSV has good stability under stress condition as well.

**Table 5**

| Condition | Time | Crystalline form | Purity (%) |
|---|---|---|---|
| Initial | - | Form CSV | 99.51 |
| 25 °C/60%RH | 6 months | Form CSV | 99.52 |
| 40 °C/75%RH | 6 months | Form CSV | 99.50 |
| 60 °C/75%RH | 2 months | Form CSV | 99.54 |

### Example 5 Stability of Form CSV under mechanical force

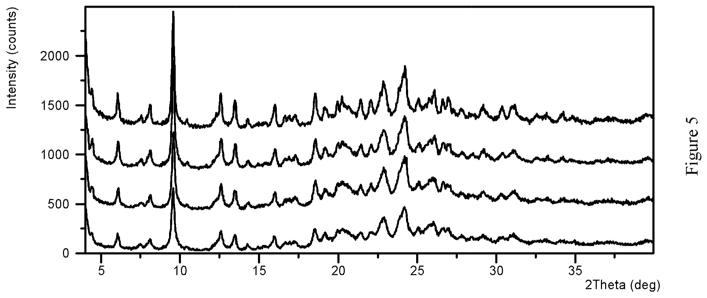

An appropriate amount of Form CSV was compressed into tablets under different pressures with a Φ6mm round tooling punch. XRPD tests were conducted before and after compression. The test results are listed in Table 6, and the XRPD overlay is depicted in Figure 5. The results indicate that Form CSV has good stability under different pressures.

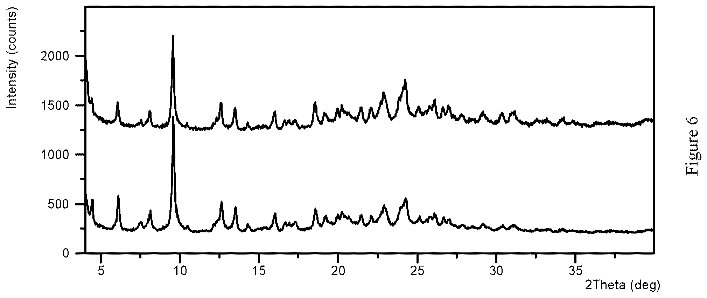

An appropriate amount of Form CSV was subjected to ball milling at 500 rpm for 5 minutes using a ball mill. XRPD tests were conducted before and after milling, and the XRPD overlay is shown in Figure 6. The results show that Form CSV remained unchanged before and after ball milling, indicating Form CSV has good stability under mechanical force.

**Table 6**

| Before tableting | Pressure | After tableting |
|---|---|---|
| Form CSV | 5 kN | Form CSV |
| | 10 kN | Form CSV |
| | 20 kN | Form CSV |

### Example 6 Preparation of Form CSVI

0.64 g of Compound I dihydrobromide solid was weighed and dissolved in 8 mL of methanol. 45 mL of toluene was added to the resulting clear solution. The mixture was magnetically stirred at -20 °C for about 18 h to induce precipitation. The resulting suspension was filtered at room temperature. The wet cake was dried at 35 °C, followed by vacuum drying at 80 °C for about 9.5 h to afford a crystalline solid. The crystalline solid was confirmed to be Form CSVI, and its XRPD pattern is substantially as depicted in Figure 7.

### Example 7 Preparation of Form CSVI

200 mg of Compound I dihydrobromide solid was weighed and dissolved in 2.5 mL of methanol. 12.5 mL of toluene was added to the resulting clear solution, followed by addition of a small amount of seed crystals. The mixture was allowed to stand at -20 °C for about 16 h for crystallization. The resulting suspension was filtered at room temperature. The wet cake was dried at 25 °C for about 4 h, followed by vacuum drying at 70 °C with water compensation for about 6 h to obtain a crystalline solid.

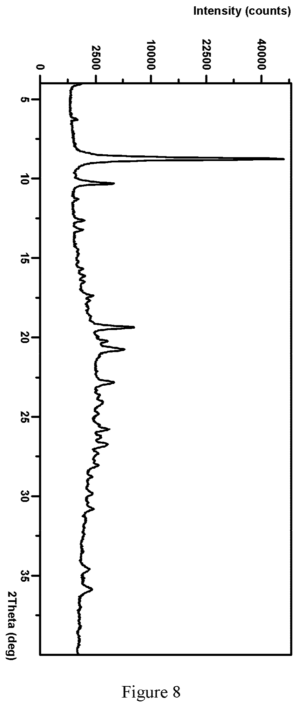

The obtained crystalline solid was confirmed to be Form CSVI, and its XRPD pattern is substantially as depicted in Figure 8.

The TGA curve of Form CSVI is depicted in Figure 9, which shows a weight loss of 1.1% when heated to 80 °C. An additional weight loss of approximately 0.6% was observed between 80 °C to 110 °C, corresponding to 0.25 molar equivalent of crystalline water.

The ¹H NMR data of Form CSVI are as follows: ¹H NMR (400 MHz, DMSO-d6) δ 11.29 (s, 1H), 9.30 (brs, 2H), 7.93 (brs, 2H), 7.75 (s, 1H), 7.54 (s, 1H), 7.08 (td, J = 9.8, 2.4 Hz, 1H), 6.90 (d, J = 9.3 Hz, 1H), 4.22 (s, 1H), 3.51 (s, 2H), 3.30 - 3.19 (m, 2H), 3.00 - 2.88 (m, 1H), 2.87 - 2.69 (m, 2H), 2.61 - 2.52 (m, 2H), 2.29 - 2.14 (m, 1H), 2.04 - 1.73 (m, 3H), 1.64 (d, J = 7.6 Hz, 6H), 1.41 - 1.25 (m, 2H), 0.91 (t, J = 7.3 Hz, 3H), 0.85 (s, 9H). The NMR results indicate that there is no residual solvent in Form CSVI.

### Example 8 Preparation of Form CSVI

200 mg of Compound I dihydrobromide solid was weighed and dissolved in 2.5 mL of methanol. 12.5 mL of toluene was added to the resulting clear solution, followed by the addition of a small amount of seed crystals. The mixture was allowed to stand at -20 °C for about 16 h to induce precipitation. The resulting suspension was filtered at room temperature. The wet cake was dried at 25 °C for about 4 h, followed by vacuum drying at 70 °C with water compensation for about 6 h. The obtained solid was then subjected to a humidity cycling treatment of 0%-60%-0%RH to yield a crystalline solid.

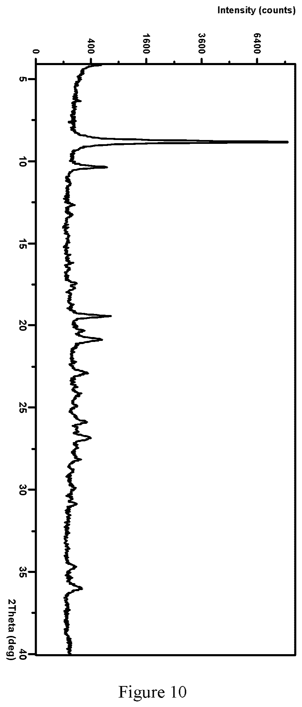

The obtained crystalline solid was confirmed to be Form CSVI. The XRPD pattern is substantially as depicted in Figure 10, and the XRPD data are listed in Table 7.

**Table 7**

| 20 (°) | d-spacing (Å) | Intensity (%) |
|---|---|---|
| 6.3 | 14.0 | 0.5 |
| 8.8 | 10.0 | 100.0 |
| 10.4 | 8.5 | 6.3 |
| 12.7 | 7.0 | 0.7 |
| 13.3 | 6.7 | 0.5 |
| 14.7 | 6.0 | 0.3 |
| 16.2 | 5.5 | 0.5 |
| 17.4 | 5.1 | 1.1 |
| 17.7 | 5.0 | 1.0 |
| 19.4 | 4.6 | 7.5 |
| 20.3 | 4.4 | 2.3 |
| 20.9 | 4.3 | 5.6 |
| 22.9 | 3.9 | 2.7 |
| 24.2 | 3.7 | 1.4 |
| 24.9 | 3.6 | 1.2 |
| 25.9 | 3.4 | 2.5 |
| 26.8 | 3.3 | 3.1 |
| 28.2 | 3.2 | 1.6 |
| 28.8 | 3.1 | 0.7 |
| 29.9 | 3.0 | 0.9 |
| 30.9 | 2.9 | 1.1 |
| 34.7 | 2.6 | 1.0 |
| 36.0 | 2.5 | 1.7 |

### Example 9 Preparation of Form CSVI

0.1934 g of Compound I dihydrobromide solid was weighed and dissolved in 2.5 mL of methanol. 12.5 mL of toluene was added to the resulting clear solution. The mixture was allowed to stand at -20 °C for about 16 h to induce precipitation. The resulting suspension was filtered at room temperature. The wet filter cake was dried at 35 °C for about 6 h, followed by vacuum drying with water compensation at 60 °C for about 3 h to obtain the crystalline solid. The obtained crystalline solid was confirmed as Form CSVI of the present disclosure.

### Example 10 Solubility of Form CSVI

When solubility test is used to predict the in vivo performance of a drug, it is critical to simulate in vivo conditions as closely as possible. For oral drugs, FaSSIF and FeSSIF can be used to simulate the conditions in vivo and predict the effect of eating. Solubilities in these media are closer to those in vivo.

Appropriate amounts of Form CSVI were suspended in FaSSIF and FeSSIF at 37 °C. After equilibration for 0.5 h, the suspensions were filtered and the concentration (mg/mL) of Compound I in each filtrate was measured by HPLC. The results are listed in Table 8, indicating that Form CSVI shows higher solubility than the prior art Form A in both FaSSIF and FeSSIF.

**Table 8**

| Medium | Form A in the prior art (mg/mL) | Form CSVI (mg/mL) |
|---|---|---|
| FaSSIF | 4.2 | 16.1 |
| FeSSIF | 7.5 | 10.1 |

### Example 11 Compressibility of Form CSVI

Tablets were prepared by an ENERPAC manual tablet press. About 60 mg of Form CSVI and the prior art Form A were separately added to Φ6mm round tooling punches and compressed into tablets under a pressure of 3 kN. The tablets were left at RT for 24 h to allow complete elastic recovery. The diameter (D) and thickness (L) were measured using a vernier caliper, and their radial crushing strength (hardness, H) was determined using a tablet hardness tester. The tensile strength (T) of the powders was calculated with the following formula: T=2H/πDL. The test results show that the average tensile strength of Form CSVI was 0.907 MPa, while that of the prior art Form A was 0.37 MPa. Under a certain force, higher tensile strength indicates better compressibility. The results indicate Form CSVI exhibits superior compressibility compared to the prior art Form A.

### Example 12 Stability of Form CSVI

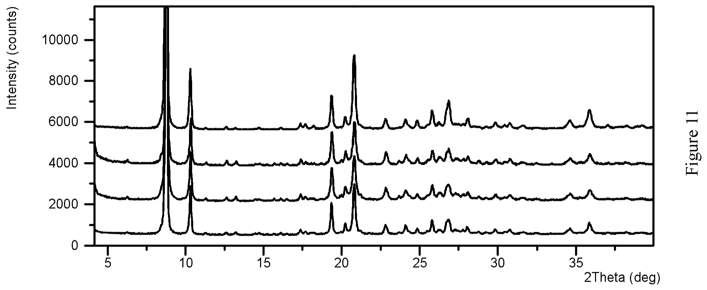

An appropriate amount of Form CSVI was packaged in a sealed container and then stored under the conditions of 25 °C/60%RH, 40 °C/75%RH and 60 °C/75%RH, respectively. Crystalline form was analyzed by XRPD. The results are listed in Table 9, and the XRPD pattern overlay is depicted in Figure 11. The results show that Form CSVI remains stable for at least 8 months under 25 °C/60%RH and 40 °C/75%RH conditions, indicating Form CSVI has good stability under long-term and accelerated conditions. Meanwhile, Form CSVI remains stable for at least 2 months under 60 °C/75%RH condition, indicating Form CSVI has good stability under stress condition as well.

**Table 9**

| Condition | Time | Solid form |
|---|---|---|
| Initial | - | Form CSVI |
| 25 °C/60%RH | 8 months | Form CSVI |
| 40 °C/75%RH | 8 months | Form CSVI |
| 60 °C/75%RH | 2 months | Form CSVI |

### Example 13 Stability of Form CSVI under mechanical force

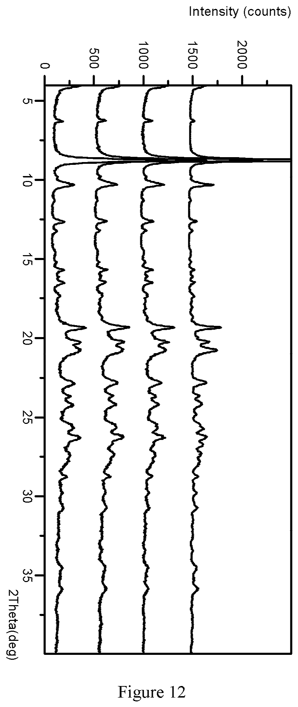

An appropriate amount of Form CSVI was compressed into tablets under different pressures with a Φ6mm round tooling punch. XRPD tests were conducted before and after compression. The test results are listed in Table 10, and the XRPD overlay is depicted in Figure 12. The results indicate that Form CSVI has good stability under different pressures.

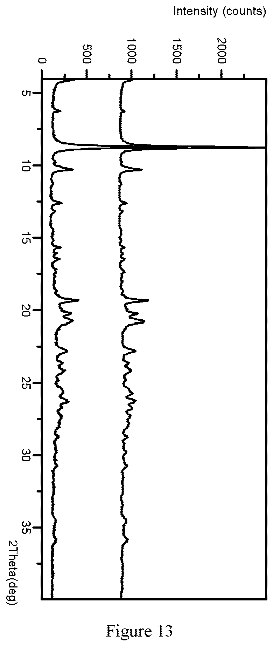

An appropriate amount of Form CSVI was subjected to ball milling at 500 rpm for 5 minutes using a ball mill. XRPD tests were conducted before and after milling, and the XRPD overlay is shown in Figure 13. The results show that Form CSVI remained unchanged before and after ball milling, indicating Form CSVI has good stability under mechanical force.

**Table 10**

| Before tableting | Pressure | After tableting |
|---|---|---|
| Form CSVI | 5 kN | Form CSVI |
| | 10 kN | Form CSVI |
| | 20 kN | Form CSVI |

The examples described above are only for illustrating the technical concepts and features of the present disclosure, and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. Crystalline form CSV of Compound I dihydrobromide, wherein the X-ray powder diffraction pattern comprises characteristic peaks at 2theta values of 6.1°±0.2°, 9.6°±0.2° and 13.6°±0.2° using Cu-Kα radiation,

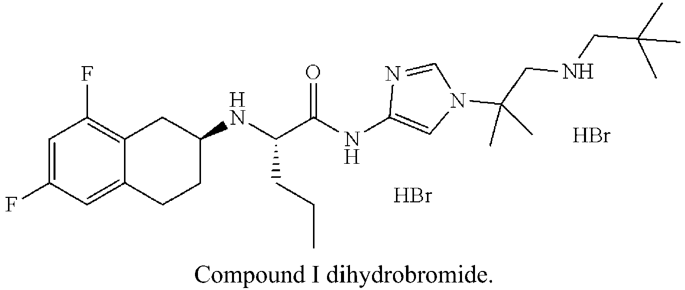

2. The crystalline form CSV of Compound I dihydrobromide according to claim 1, wherein the X-ray powder diffraction pattern comprises at least one characteristic peak at 2theta values of 4.5°±0.2°, 16.1°±0.2° and 18.6°±0.2° using Cu-Kα radiation.

3. The crystalline form CSV of Compound I dihydrobromide according to claim 1, wherein the X-ray powder diffraction pattern comprises at least one characteristic peak at 2theta values of 12.7°±0.2°, 21.5°±0.2° and 23.0°±0.2° using Cu-Kα radiation.

4. The crystalline form CSV of Compound I dihydrobromide according to claim 2, wherein the X-ray powder diffraction pattern comprises at least one characteristic peak at 2theta values of 12.7°±0.2°, 21.5°±0.2° and 23.0°±0.2° using Cu-Kα radiation.

5. The crystalline form CSV of Compound I dihydrobromide according to claim 1, wherein the X-ray powder diffraction pattern is substantially as depicted in Figure 1 using Cu-Kα radiation.

6. Crystalline form CSVI of Compound I dihydrobromide, wherein the X-ray powder diffraction pattern comprises characteristic peaks at 2theta values of 8.8°+0.2°, 10.4°±0.2°, 19.4°±0.2° and 20.9°±0.2° using Cu-Kα radiation,

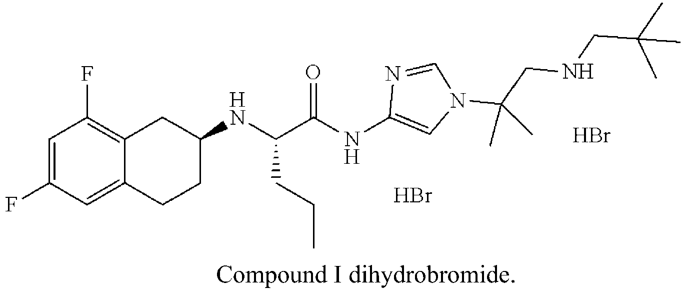

7. The crystalline form CSVI of Compound I dihydrobromide according to claim 6, wherein the X-ray powder diffraction pattern comprises at least one characteristic peak at 2theta values of 22.9°±0.2°, 26.8°±0.2° and 25.9°±0.2° using Cu-Kα radiation.

8. The crystalline form CSVI of Compound I dihydrobromide according to claim 6, wherein the X-ray powder diffraction pattern is substantially as depicted in Figure 7 or Figure 8 or Figure 10 using Cu-Kα radiation.

9. A pharmaceutical composition, wherein said pharmaceutical composition comprises a therapeutically effective amount of crystalline form CSV of Compound I dihydrobromide according to claim 1, or crystalline form CSVI of Compound I dihydrobromide according to claim 6, or combinations thereof, and pharmaceutically acceptable excipients.

10. Crystalline form CSV of Compound I dihydrobromide according to claim 1, or crystalline form CSVI of Compound I dihydrobromide according to claim 6, or combinations thereof for use in preparing drugs of γ-secretase inhibitor.

11. Crystalline form CSV of Compound I dihydrobromide according to claim 1, or crystalline form CSVI of Compound I dihydrobromide according to claim 6, or combinations thereof for preparing drugs for treating desmoid tumors.
